# EUROPEAN PATENT SPECIFICATION

(11) **EP 1 293 225 B1**
(45) Date of publication and mention of the grant of the patent: **08.08.2007**
(21) Application number: 02020687.6
(22) Date of filing: 13.09.2002
(51) Int. Cl.: A61M 15/00

(54) **Dry powder inhaler**
Trockenpulverinhalator
Inhalateur à poudre sèche

(30) Priority: 14.09.2001 JP 2001278974
(43) Date of publication of application: 19.03.2003
(73) Proprietor: Omron Healthcare Co., Ltd., Kyoto 615-0084 (JP); Takano, Hiroshi, Kyotanabe, Kyoto 610-0321 (JP)
(72) Inventor: Takano, Hiroshi, c/o Doshisha University, Kyotanabe-shi, Kyoto 610-0321 (JP); Asai, Kei, c/o Omron Corp., 801, Minamifudodo-cho, Kyoto-shi, Kyoto 600-8530 (JP); Tabata, Makoto, c/o Omron Healthcare Co. Ltd., Kyoto 615-0084 (JP)
(74) Representative: Wilhelms . Kilian & Partner Patentanwälte

(56) References cited:
- US-A- 3 858 583
- US-A- 3 971 377
- US-A- 4 338 931
- US-A- 4 627 430
- US-A- 5 577 497

## Description

### Background of the Invention

This invention relates to a module of drug particle separator for forming dry powder in an inhaler used by a patient of a respiratory disease for inhaling a medicament, as well as an inhaler provided with such a module.

Dry powder inhalers for assisting a patient of a respiratory disease such as asthma in inhaling a medicament to control or improve the condition of the disease are becoming commercially available. A dry powder medicament is usually formed by attaching 1-10µm of effective component (medicament) to several tens to about 100µm of lactose as a so-called diluent base for making the dry powder medicament more easily ingestible. A dry powder inhaler is adapted to operate by the inhalation of the patient, generating a turbulent flow of air as the patient inhales and causing the medicament to become separated from the lactose such that the patient can inhale the separated medicament.

One of the problems with such a prior art inhaler is that the degree of turbulent flow generation depends heavily on the speed of inhaled air. If the patient is very sick or very young and a sufficient air speed cannot be obtained, there may not be enough turbulent flow of air with the respiration and the medicament may not become separated sufficiently. Since lactose with the medicament remaining attached has a relatively large momentum of inertia, it may collide with the throat. Since its diameter is relatively large, furthermore, it may become attached to the upper respiratory tracts such that the medicament may fail to reach a deep part of the lung where it is destined.

Another problem is that medicaments are not always spherical. Some of them may be elongated or needle-shaped. Since the medicament particles generated from a commercially available dry powder inhaler are oriented in various angles, their mass medium aerodynamic diameters are not constant but have a large distribution. As a result, the distribution is also sufficiently large in the position inside the patient's body where the medicaments deposit and this makes the therapeutic effect on the patient difficult to predict.

Still another problem with the prior art dry powder inhaler is its inability to select a distribution of diameters of particles to be emitted.

From US-A-4627430 an inhaler is known which includes a container for holding a liquid inhalant into which a suction cone is submerged for sucking the liquid inhalant from the container and nebulizing the inhalant.

US-A- 397 1 377 discloses an inhaler showing the features of the preamble of claim 1.

### Summary of the Invention

It is therefore an object of this invention to provide an improved dry powder inhaler capable of effectively separating medicament from lactose, independent of the rate of inhalation by the patient, making the orientation of medicament particles uniform and varying the distribution of medicament diameters.

A dry powder inhaler according to this invention is as defined in claim 1, characterized not only as comprising a structure providing a flow route for medicament from an upstream side to a downstream side so as to be synchronized with the respiration, a rotor placed in this flow route and a driving means for rotating the rotor, but also wherein the rotor has a convex surface on the upstream side. The convex surface of the rotor on the upstream side may include a portion which is conical or semispherical or a portion of a cone or a sphere. In other words, the rotor surface may be of the shape of a cone with its tip portion removed.

By providing an inhaler with a separator thus structured, a steady-state flow is generated near the rotary cone as the rotor is rotated and a shearing force operates on the medicament distributed in this flow, caused by the speed differentiation on the particles within the medicament. Thus, only the medicament distributed from a supply source is easily separated from lactose as they pass by the rotor. As a result, the medicament particles can be dependably delivered to a specified body part of the patient requiring treatment such as a deep interior of the lung.

While the medicament particles separated from lactose are floating near the sloped surface of the rotating rotor, they come to be orientated in a same direction if they are of a flat shape or a needle-like shape. Since their aerodynamical particle diameters become uniform, they can be more efficiently deposited at a target position inside the patient.

If a fan is disposed on the upstream side of the rotor for dispersing and sending the medicament towards the rotor, the medicament particles are forcibly accelerated towards the rotor and hence can be more effectively separated from lactose.

A module of the drug particle separator according to a preferred embodiment of the invention is further provided with a detector for detecting the rotational speed of the rotor and a control unit for controlling the driving means according to the rotational speed of the rotor detected by the detector. With such a detector and a control unit, the drug particle separator can be controlled in various different ways according to the results detected by the detector. For example, the rotary speed (the number of rotations per unit time) of the rotor may be adjusted so as to vary the distribution of the diameters of the medicament particles. As another example, the rotation of the rotor may be controlled according to the measured breathing speed of the patient. In such an application, medicament particles can be generated so as to have a size which is not dependent upon the breathing speed of the patient. The rotor may be controlled to start its rotation in synchronism with the breathing of the patient.

If the rotor and the fan are attached to a same rotary shaft, they can be operated by a single driving means, and hence the number of parts to be assembled can be reduced and the module can be made smaller. If the driving means is further arranged coaxially with the rotor and the fan, the series of operations in an airflow including the supply of medicament particles from a source, their separation from lactose and transportation of the separated medicament can be carried out efficiently.

### Brief Description of the Drawings

Fig. 1 is an external view of an inhaler embodying this invention.
Fig. 2 is an exploded view of the inhaler of Fig. 1.
Fig. 3 is a sectional view of the inhaler of Fig. 1.
Fig. 4 is a side view of a conically shaped rotor for showing the dimensions of its examples.
Fig. 5 is a diagram for showing the shearing force on medicament particles inside the inhaler of this invention.
Figs. 6A and 6B are drawings for explaining the shearing force which acts on medicament particles due to the rotary motion of the rotor of the inhaler.
Fig. 7 is a sectional view of another inhaler embodying this invention.
Fig. 8 is an exploded diagonal view of still another inhaler embodying this invention.
Fig. 9 is a sectional view of the inhaler of Fig. 8.
Fig. 10 is a schematic block diagram of the inhaler of Figs. 8 and 9.
Fig. 11 is a graph showing the relationship between the rotational speed of the rotor and the particle diameter of medicament and its distribution.
Fig. 12 is a sectional view of a portion of still another inhaler embodying this invention.
Fig. 13 is a schematic block diagram of the inhaler of Fig. 12.
Figs. 14-1, 14-2, 14-3, 14-4, 14-5 and 14-6, together referred to as Fig. 14, are side views of examples of rotors with different shapes which may be used in the inhaler of this invention.

### Detailed Description of the Invention

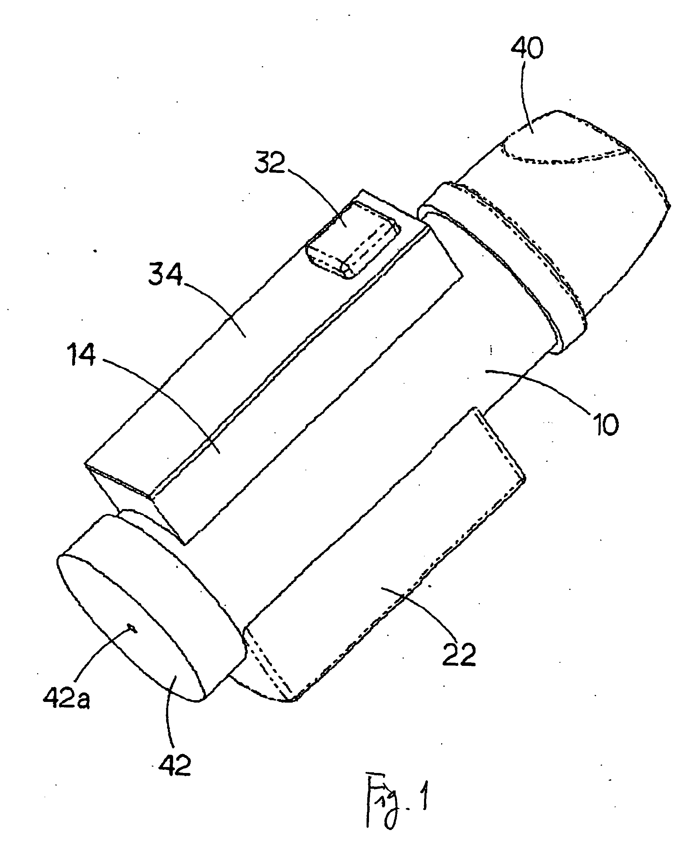

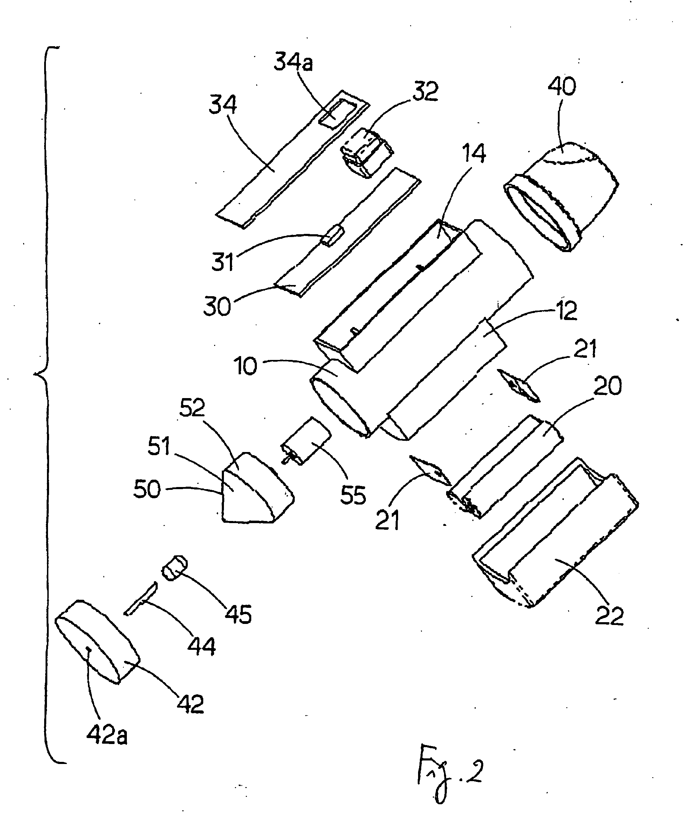

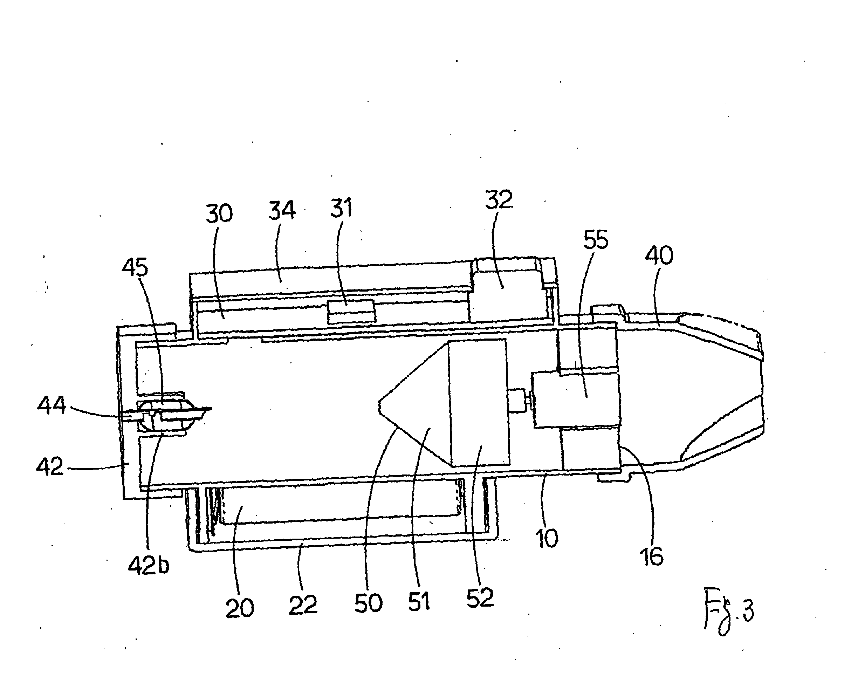

The invention is described next with reference to an example. Fig. 1 is an external view of an inhaler embodying this invention. Fig. 2 is its exploded view and Fig. 3 is its sectional view. As shown, the inhaler has a tubular case 10 forming inside a flow route for medicament and includes a battery storage section 12 capable of containing two batteries 20 therein. Battery terminals 21 are provided on both sides of the battery storage section 12 and a battery cover 22 is detachably attached thereto.

On the opposite side of the case 10 is a circuit container 14 containing therein a baseboard 30 having mounted thereonto a CPU 31 serving as a control means as well as a switch 32 and being provided with a cover 34. The switch 32 is exposed to the exterior, penetrating the cover 34 through a throughhole 34a therethrough.

A mouthpiece 40 is disengageably engaged to an opening at one end of the case 10 and a bottom cover 42 is engaged to and closes the opposite opening of the tubular case 10. At the center of the bottom cover 42 is an air hole 42a into which is inserted a partially open needle 44, penetrating a capsule 45 containing medicament such that the opening of the needle 44 is situated inside the capsule 45. The capsule 45 is supported by being pushed into a supporting frame 42b protruding inward from the bottom cover 42.

A rotor 50 having a conically shaped surface towards the capsule 45 is rotatably supported inside the case 10. According to this example, the rotor 50 has a conical portion 51 with a sloped outer surface and a circular disk-shaped base portion 52. A small gap is maintained between the outer peripheral surface of the base portion 52 and the inner surface of the case 10. The shaft of a rotor motor (or driving means) 55 for causing the rotor 50 to rotate is fastened to the center of the base portion 52 of the rotor 50. The motor is affixed to the center of an attachment member 16 formed inside the case 10 such that gaps are provided to the attachment member 16 for allowing air and medicament to flow through. The rotary shaft of the motor 55, the central axis of the rotor 50 and the needle 44 are arranged to be in a mutually coaxial relationship inside the case 10.

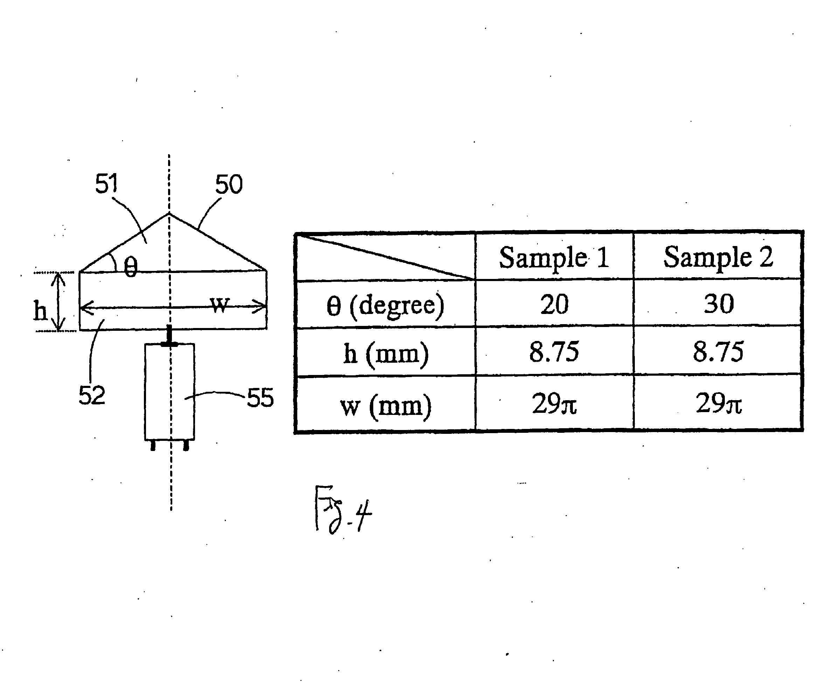

The size and shape of the rotor 50 may depend on the size of the inhaler. Fig. 4 shows two examples of rotors with a conically shaped portion 51 in terms of its slope θ with respect to the base portion 52, the height h of the base portion 52 and the outer diameter w of the base portion 52. Example 1 has θ = 20°, Example 2 has θ = 30°, h and w being the same for both Examples 1 and 2.

When the switch 32 is pressed to switch on the motor 55, the rotor 50 begins to rotate, If a patient breathes in through the mouthpiece 40, a negative pressure is generated inside the case 10, causing air to flow in through the air hole 42a in the bottom cover 42 and the needle 44 to the interior of the case 10. As the air passes through the needle 44, the medicament inside the capsule 45 is sucked in through the opening of the needle 44 and dispersed inside the case 10. The dispersed medicament is then caused to move towards the rotor 50.

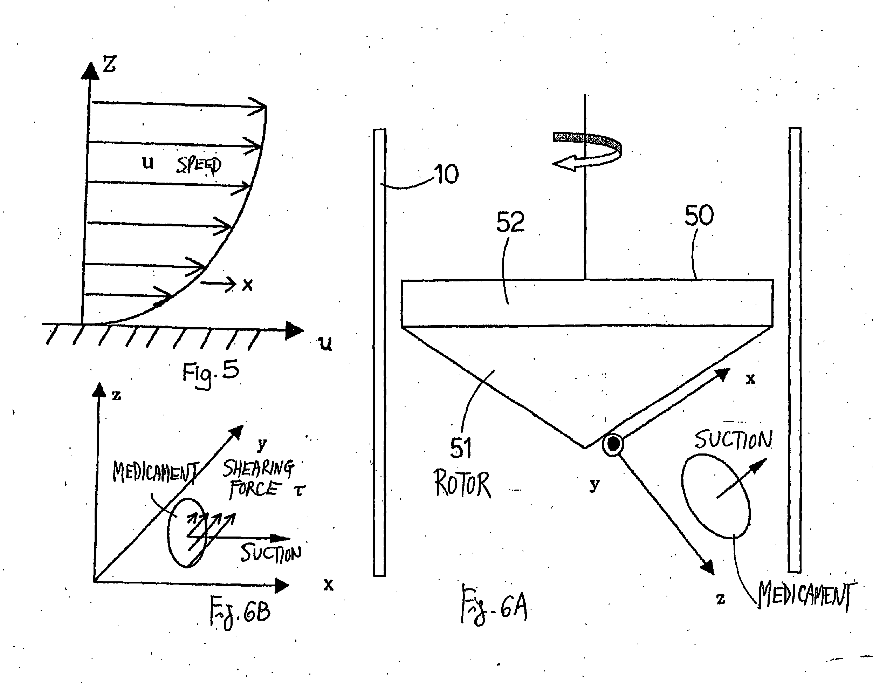

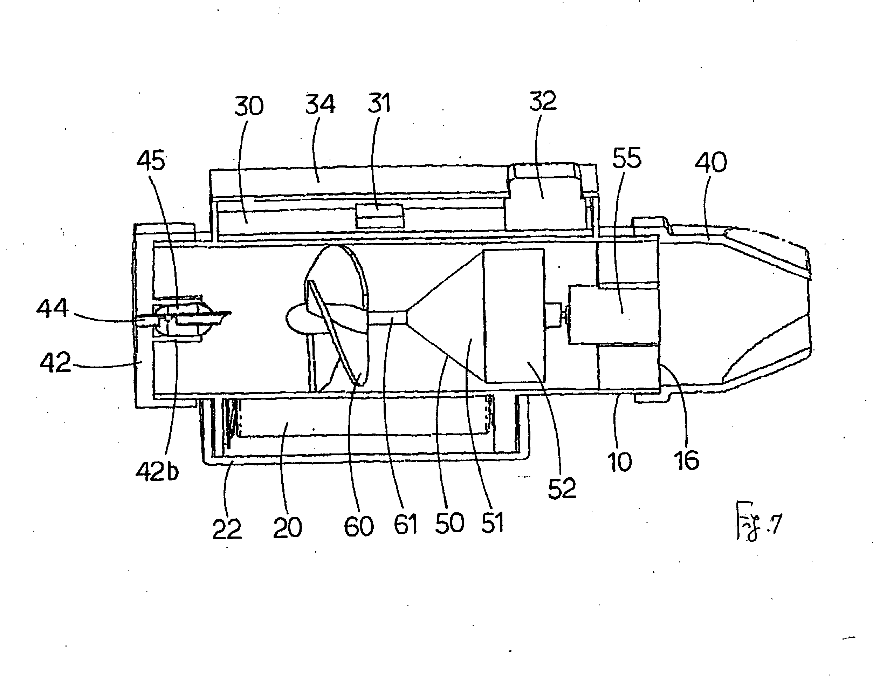

In the meantime, there is a steady-state flow of air developed near the peripheral surface of the conical portion 51 due to the rotary motion of the rotor 50. As a result, the medicament distributed within this steady-state flow experiences a shearing force developed by the speed differentiation generated on the medicament. In general, such a shearing force is proportional to the slope in the speed u or the rate at which speed of the steady-state flow changes. As shown in Fig. 5, if an x-axis is chosen along the surface of an object in the direction of a steady-state flow and a z-axis defined perpendicular to the surface, the shearing force τ at each point is shown as τ = η (du/dz) wheres η is the viscosity. Fig. 6A shows a coordinate system defined similarly on the surface of the conical portion 51 of the rotor 50 shown in Figs. 1-3, the z-axis being perpendicular to the conical surface, the x- and y-axes being tangent thereto, as shown. In this case, as shown in Fig. 6B, a suction force results in the x-direction and a shearing force τ results in the y-direction. Lactose and the medicament are separated by this shearing force.

Since the inhaler is usually used in an upright position with the mouthpiece 40 oriented upward, the separated lactose drops downward by the force of gravity and only the medicament in powder form is sucked in the direction of the mouthpiece 40. Thus, the medicament can be caused to dependably reach the position of treatment such as a deep interior of the lung and its therapeutic effect can be improved. Moreover, while the medicament is floating near the rotating sloped surface of the rotor 50, needle-shaped crystalline particles and flat-shaped particles will come to float at a fixed angle, and the medicament particles come to possess an aerodynamically uniform particle size. This has the favorable result of effectively causing the medicament to be deposited within the patient's body.

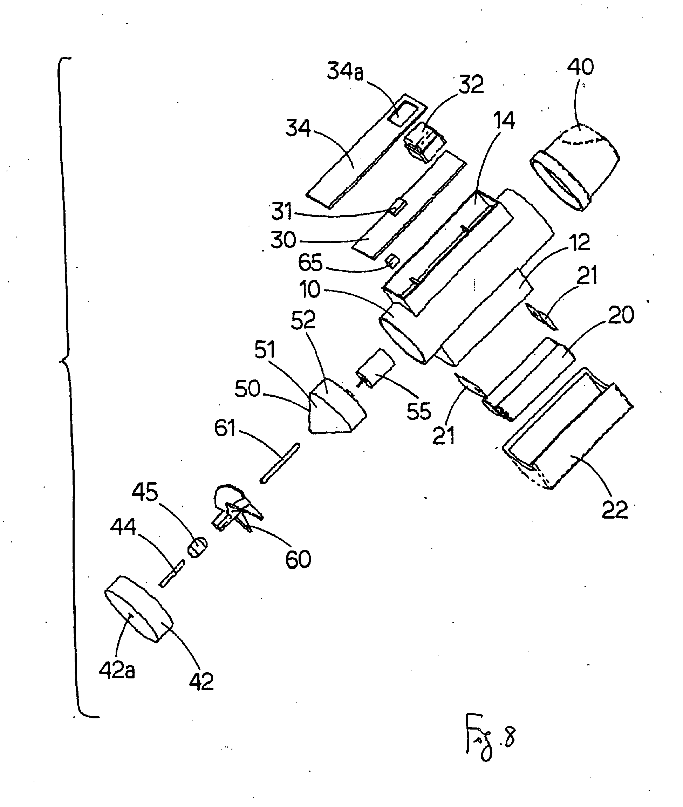

Fig. 7 shows another inhaler according to another embodiment of the invention different from the inhaler described above with reference to Figs. 1-3 only wherein a fan 60 is provided within the case 10 for dispersing and accelerating the medicament towards the rotor 50. The fan 60 is attached to a shaft 61 which is in turn attached to the rotor 50 at its center between the capsule 45 and the rotor 50 inside the case 10 so as to be coaxial with the rotor 50 and to rotate together with the rotor 50. The fan 60, thus rotating with the rotor 50, serves to forcibly transmit the medicament separated from the capsule 45 towards the sloped surface of the conical portion 51 of the rotor 50. Thus, the medicament is more effectively separated from lactose. Since the rotor 50, the fan 60 and the motor 55 are arranged coaxially, the space inside the case 10 is efficiently utilized and the separation and emission of medicament can be carried out efficiently in one airflow.

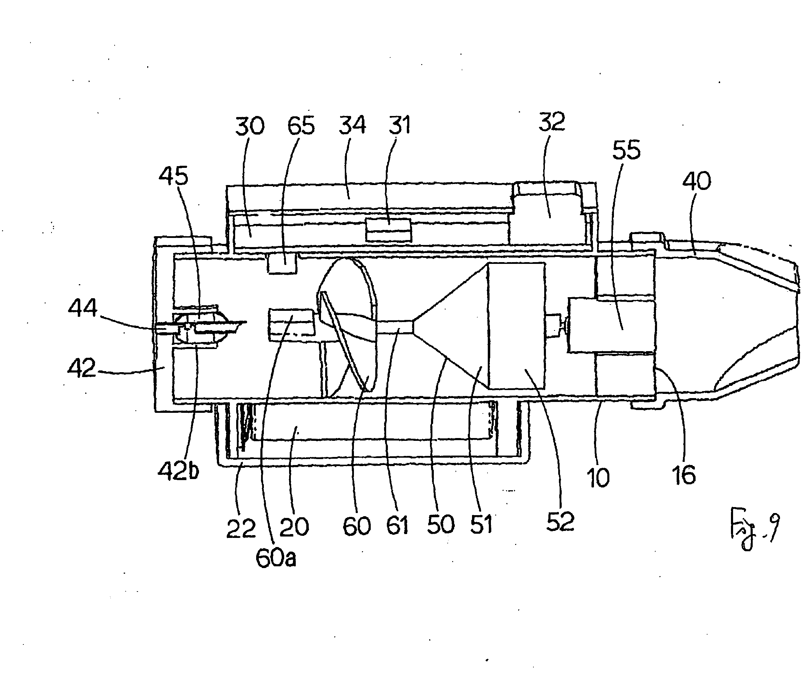

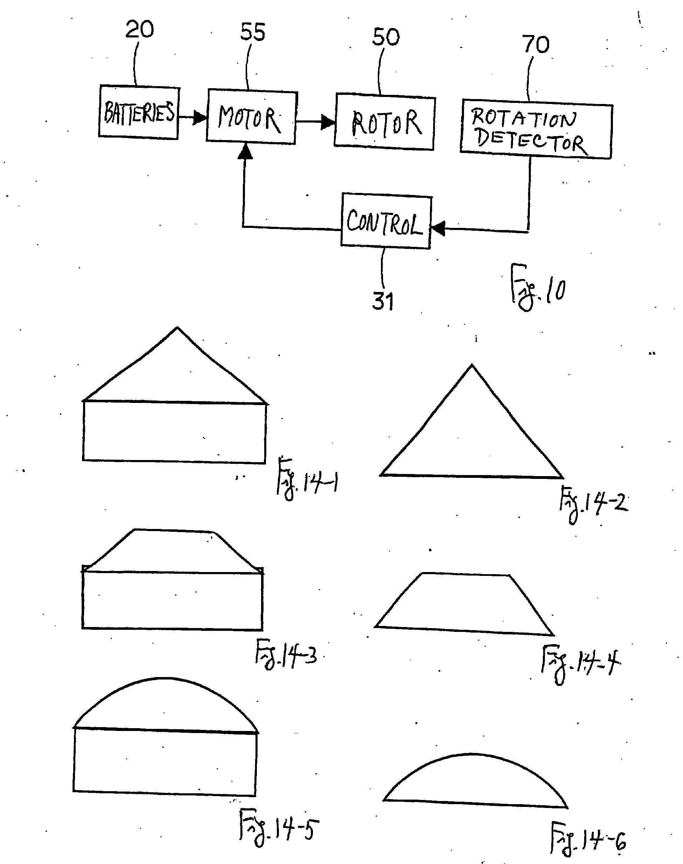

Figs. 8 and 9 show still another inhaler embodying this invention which is structured similar to the inhaler described above with reference to Fig. 7 but additionally comprises a photosensor 65 for detecting the rotational speed of the rotor 50. In the illustrated example, the photosensor 65 is of a so-called reflected light type, having a target member 60a attached to the tip of the fan 60 to be detected by the photosensor 65. The photosensor 65 is disposed on the back side of the base board 30 and a black line is painted on the target member 60a opposite the photosensor 65 such that the rotation of the fan 60 and hence that of the rotor 50 can be thereby detected. The photosensor 65 and the target member 60a together form a rotation detector 70 (shown in Fig. 10).

A method of operating this inhaler is schematically shown by a block diagram in Fig. 10. The rotational speed of the rotor 50 detected by the photosensor 65 is transmitted to the control unit (CPU 31) which serves to carry out a feedback control on the motor 55.

The rotary motion of the rotor 50 may be controlled in different manners. For example, the rotational speed of the rotor 50 may be controlled so as to vary the distribution of particle size of the medicament. In such a mode of operation, medicament with particle diameter not dependent upon the patient's breathing speed may be generated. Fig. 11 is a graph showing the relationship between the rotational speed of the rotor 50 and the particle diameter of medicament and its distribution. As can be seen in Fig. 11, if the rotor 50 is not rotating (or if the rotor is not provided), the distribution of the medicament particles is centered around about 7µm but the distribution comes to center around about 5µm if the rotor 50 rotates at the rate of 7000rpm and around about 2µm if the rotor 50 rotates at the rate of 10000rpm. Recent experiments by the inventors herein show that the distribution is centered around about 1µm if the rotor rotates at the rate of 12000rpm. Thus, if the rotor 50 is rotated at a fast rate over 10000rpm, the medicament particles may be made uniform to a mass medium aerodynamic diameter of about 1µm such that they can efficiently reach a deep part of the patient's body, although it is not desirable to reduce the mass medium aerodynamic diameter to less than 1µm because such medicament particles may reach a deep therapy-requiring part of the patient's body but may not be deposited there successfully and may be-breathed out of the body. A desired distribution of particle size can be generated by controlling the rotational speed of the rotor 50 independent of the breathing speed of the patient.

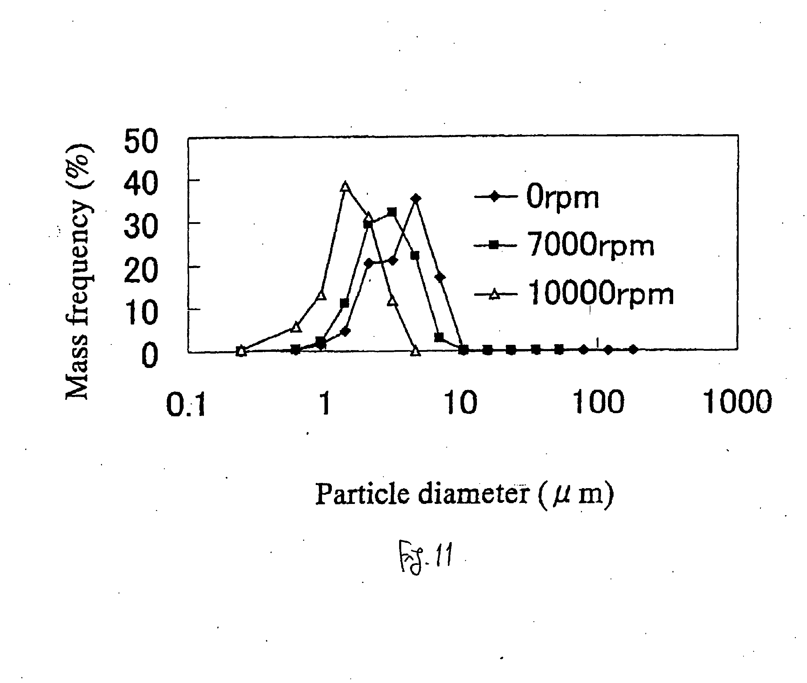

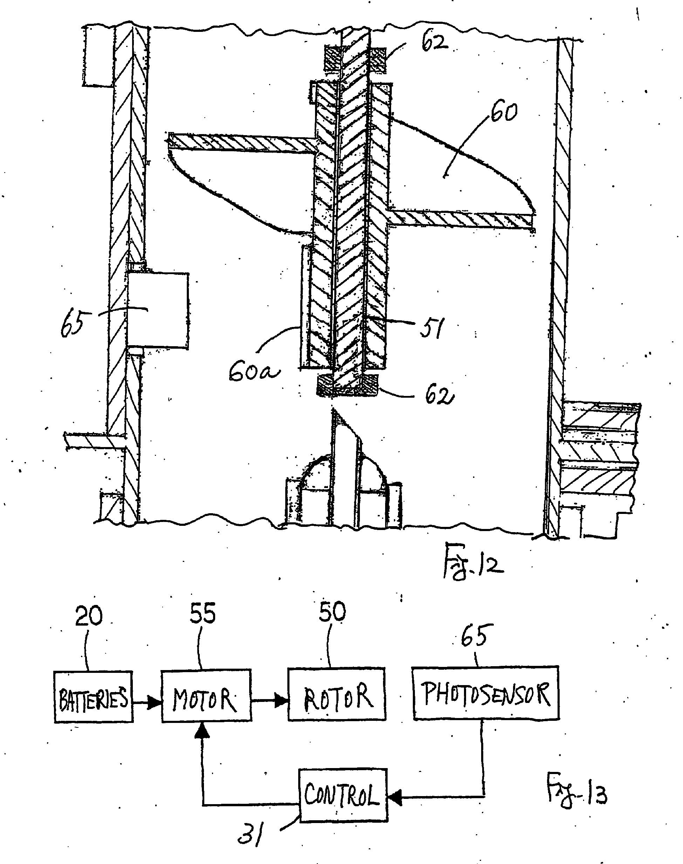

Figs. 12 and 13 show an inhaler according to still another embodiment of the invention, which is similar to the one described above with reference to Figs. 8 and 9 but is characterized wherein its fan 60 and the rotor (and hence its drive shaft 51) are adapted to rotate independently of each other. As a patient breathes through the mouthpiece (as shown at 40 in Figs. 8 and 9 but not shown in Fig. 12), the fan 60 undergoes a rotary motion according to the amount of respiration. A target member 60a for detection is attached to the fan 60, and the rotary motion of the fan 60, representing the air suction speed, can be detected by a photosensor 65 (say, of a so-called reflected light type) serving as a suction air speed sensor

As shown in Fig. 13, the air suction speed determined by the photosensor 65 is transmitted to a control unit (with a CPU 31) and compared with a preliminarily stored specified speed. If the detected air suction speed is found to exceed this specified speed, the control unit 31 judges that the patient's respiration is sufficiently strong and activates the motor 55 for the rotor 50, causing the rotor 50 to rotate at a specified speed such as 10000rpm. In Fig. 12, numerals 62 indicate stoppers for limiting the movement of the fan 60 along the drive shaft 51 of the motor 50 and numeral 51 indicates a bearing inserted between the shaft for the fan 60 and the drive shaft 51 of the motor 50 for allowing their mutually independent rotary motions.

The control unit (or the CPU 31) may be programmed so as to control the rotary motion (speed) of the rotor 50 according to the air suction speed by the patient. When the rate of suction by the patient is determined to be greater than a specified rate, the measured suction rate may be divided into several steps. In general, the smaller the mass medium aerodynamic diameter of a medicament, the deeper it becomes likely for it to be able to reach in the patient's body. Since medicament particles have a mass, they acquire an inertial force, or momentum, as they begin to move. Larger medicament particles have a larger momentum and cannot change the direction of motion easily. They tend to collide with the inner wall of the trachea and it becomes difficult for them to reach a deeper part of the lung. While the air suction speed is relatively low, relatively large medicament particles may be able to reach a deeper part of the lung even if the rotor 50 is rotated at a relatively low speed such as 7000rpm. As the air suction speed is increased, the motion of the rotor 50 may be increased, say, to 10000rpm so as to reduce the diameters of the medicament particles such that they can reach deeper into the lung as when the air suction speed was lower.

The rotary speed of the rotor 50 need not be changed in a step-wise fashion. It may be changed continuously or linearly corresponding to the air suction speed by the patient. The rotation of the rotor 50 may be stopped as the patient stops inhaling and starts to exhale. Alternatively, the control unit (or the CPU 31) may be provided with a timer and control the rotor 50 to continue rotating for a specified length of time (such as a few seconds) once it is detected that a specified amount of air has been breathed in. The energy consumption of the batteries can be reduced by any of these control methods because the rotation of the rotor 50 is not started until the patient begins to breath in at a specified air suction rate. Rotation of the rotor 50 may also be controlled so as to start in synchronism with the breathing of the patient.

The invention has been described above with reference to only a limited number of embodiments but these illustrated embodiments are not intended to limit the scope of the invention. Many modifications and variations are possible within the scope of the invention. For example, although only rotors with a conically shaped surface have been illustrated, the rotor surface may be in many different shapes. Fig. 14 shows some of the examples, including conical shapes with a tip portion removed (such as shown in Figs. 14-3 and 14-4) or without a tip portion removed (such as shown in Figs. 14-1 and 14-2) and spherical shapes (such as shown in Figs. 14-5 and 14-6), all with a cylindrical base (such as shown in Figs. 14-1, 14-3 and 14-5) or without a cylindrical base (such as shown in Figs. 14-2, 14-4 and 14-6). All such modifications and variations that may be apparent to a person skilled in the art are intended to be within the scope of this invention.

## Claims

1. Dry powder inhaler in which an airflow is created through an inhalation action by a patient for allowing the patient to inhale a medicament, said inhaler comprising a case (10) defining a tubular flow route for a medicament from an upstream side to a down stream side of said case (10), a source of medicament (45) disposed on said upstream side and a mouthpiece (40) disposed on the downstream side,
a particle separator placed in said air flow route at a distance from said source of medicament (45), said particle separator comprising a rotor (50) driven by driving means (55), **characterized in that** said rotor has a convex surface (51) facing said source of medicament (45).

2. The inhaler of claim 1 wherein said convex surface is one selected from the group consisting of surfaces which are at least in part conical (51) and portions of a spherical surface.

3. The inhaler of claim 1 further comprising a fan (60) disposed on said upstream side of said rotor (50) for dispersing and accelerating said medicament towards said rotor.

4. The inhaler of claim 1 further comprising a detector for detecting the rotational speed of said rotor (50) and a control unit for controlling said driving means (55) according to the rotational speed of said rotor detected by said detector.

5. The inhaler of claim 3 further comprising a detector for detecting the rotational speed of said rotor (50) and a control unit for controlling said driving means (55) according to the rotational speed of said rotor detected by said detector.

6. The inhaler of claim 3 wherein said fan (60) and said rotor (50) are coaxially arranged.

7. The inhaler of claim 4 wherein said fan (60) and said rotors (50) are coaxially arranged.

8. The inhaler of claim 5 wherein said fan (60) and said rotor (50) are coaxially arranged.

9. The inhaler of claim 3 wherein said fan (60), said rotor (50) and said driving means (55) are coaxially arranged.

10. The inhaler of claim 4 wherein said fan (60), said rotor (50) and said driving means (55) are coaxially arranged.

11. The inhaler of claim 5 wherein said fan (60), said rotor (50) and said driving means (55) are coaxially arranged.

12. The inhaler of claim 1 further comprising a cover (42) with an air (42a) hole disposed upstream to said rotor (50) and a capsule (45) at said hole for containing said medicament.

13. The inhaler of claim 12 further comprising a partially open needle (44) inserted into said hole.

14. The inhaler of claim 13 wherein a driving shaft of said driving means (55), a center axis of said rotor (50) and said needle (44) are coaxially disposed.

15. The inhaler of claim 1 further comprising a suction air speed sensor (65) for measuring a suction air speed by a patient, said driving means (55) rotating said rotor (50) according to the suction air speed measured by said suction air speed sensor (65).

16. The inhaler of claim 4 further comprising a suction air speed sensor (65) for measuring a suction air speed by a patient; said control unit controlling said driving means (55) according to the suction air speed measured by said suction air speed sensor (65).

17. The inhaler of claim 5 further comprising a suction air speed sensor (65) for measuring a suction air speed by a patient; said control unit controlling said driving means (55) according to the suction air speed measured by said suction air speed sensor (65).

18. The inhaler of claim 1 wherein said rotor rotates at a rotary speed of 1000 min⁻¹ or greater.

## Patentansprüche

1. Trockenpulverinhalator, bei welchem ein Luftstrom durch eine Inhalationstätigkeit eines Patienten erzeugt wird, der es dem Patienten erlaubt, ein Medikament zu inhalieren, wobei der Inhalator ein Gehäuse (10), welches einen rohrförmigen Strömungsweg für ein Medikament von einer stromauf liegenden Seite zu einer stromab liegenden Seite des Gehäuses (10) definiert, eine Medikamentenquelle (45), die an der stromauf liegenden Seite angeordnet ist, und ein Mundstück (40), das an der stromab liegenden Seite angeordnet ist,
einen Teilchentrenner, der in dem Luftströmungsweg in einem Abstand von der Medikamentenquelle (45) angeordnet ist, aufweist, wobei der Teilchentrenner einen mit Antriebsmitteln (55) betriebenen Rotor (50) aufweist, **dadurch gekennzeichnet, dass** der Rotor eine konvexe Oberfläche (51) aufweist, die der Medikamentenquelle (45) zugekehrt ist.

2. Inhalator nach Anspruch 1, wobei die konvexe Oberfläche eine aus der Gruppe, bestehend aus Oberflächen, welche wenigstens teilweise konisch (51) sind, und Teilen einer sphärischen Oberfläche, ausgewählte ist.

3. Inhalator nach Anspruch 1, welcher ferner einen stromaufseitig des Rotors (50) angeordneten Ventilator (60) zum Zerstäuben und Beschleunigen des Medikaments zum Rotor hin aufweist.

4. Inhalator nach Anspruch 1, welcher ferner einen Detektor zur Feststellung der Drehgeschwindigkeit des Rotors (50) und eine Steuereinheit zur Steuerung der Antriebsmittel (55) gemäß der mit dem Detektor festgestellten Drehgeschwindigkeit des Rotors aufweist.

5. Inhalator nach Anspruch 3, welcher ferner einen Detektor zur Feststellung der Drehgeschwindigkeit des Rotors (50) und eine Steuereinheit zur Steuerung der Antriebsmittel (55) gemäß der mit dem Detektor festgestellten Drehgeschwindigkeit des Rotors aufweist.

6. Inhalator nach Anspruch 3, wobei der Ventilator (60) und der Rotor (50) koaxial angeordnet sind.

7. Inhalator nach Anspruch 4, wobei der Ventilator (60) und der Rotor (50) koaxial angeordnet sind.

8. Inhalator nach Anspruch 5, wobei der Ventilator (60) und der Rotor (50) koaxial angeordnet sind.

9. Inhalator nach Anspruch 3, wobei der Ventilator (60), der Rotor (50) und die Antriebsmittel (55) koaxial angeordnet sind.

10. Inhalator nach Anspruch 4, wobei der Ventilator (60), der Rotor (50) und die Antriebsmittel (55) koaxial angeordnet sind.

11. Inhalator nach Anspruch 5, wobei der Ventilator (60), der Rotor (50) und die Antriebsmittel (55) koaxial angeordnet sind.

12. Inhalator nach Anspruch 1, welcher ferner eine Abdeckung (42) mit einem stromauf des Rotors (50) angeordneten Luftloch (42a) und eine an dem Loch angeordnete Kapsel (45) zur Aufnahme des Medikaments aufweist.

13. Inhalator nach Anspruch 12, welcher ferner eine in das Loch eingesetzte teilweise offene Nadel (44) aufweist.

14. Inhalator nach Anspruch 13, wobei eine Antriebswelle der Antriebsmittel (55), eine Mittelachse des Rotors (50) und die Nadel (44) koaxial angeordnet sind.

15. Inhalator nach Anspruch 1, welcher ferner einen Saugluftgeschwindigkeitssensor (65) zur Messung einer Saugluftgeschwindigkeit durch einen Patienten aufweist, wobei die Antriebsmittel (55) den Rotor (50) gemäß der mit dem Saugluftgeschwindigkeitssensor (65) gemessenen Saugluftgeschwindigkeit drehen.

16. Inhalator nach Anspruch 4, welcher ferner einen Saugluftgeschwindigkeitssensor (65) zur Messung einer Saugluftgeschwindigkeit durch einen Patienten aufweist, wobei die Steuereinheit die Antriebsmittel (55) gemäß der mit dem Saugluftgeschwindigkeitssensor (65) gemessenen Saugluftgeschwindigkeit steuert.

17. Inhalator nach Anspruch 5, welcher ferner einen Saugluftgeschwindigkeitssensor (65) zur Messung einer Saugluftgeschwindigkeit durch einen Patienten aufweist, wobei die Steuereinheit die Antriebsmittel (55) gemäß der mit dem Saugluftgeschwindigkeitssensor (65) gemessenen Saugluftgeschwindigkeit steuert.

18. Inhalator nach Anspruch 1, wobei der Rotor mit einer Drehgeschwindigkeit von 1000 min⁻¹ oder größer rotiert.

## Revendications

1. Inhalateur à poudre sèche dans lequel un flux d'air est créé par l'intermédiaire d'une action d'inhalation d'un patient pour permettre au patient d'inhaler un médicament, ledit inhalateur comprenant un boîtier (10) définissant un trajet d'écoulement tubulaire pour un médicament depuis un côté en amont vers un côté en aval dudit boîtier (10), une source de médicament (45) disposée sur ledit côté en amont et un embout (40) disposé sur le côté en aval,
un séparateur de particules placé dans ledit trajet d'écoulement d'air à distance de ladite source de médicament (45), ledit séparateur de particules comprenant un rotor (50) entraîné par un moyen d'entraînement (55), **caractérisé en ce que** ledit rotor a une surface convexe (51) faisant face à ladite source de médicament (45).

2. Inhalateur selon la revendication 1, dans lequel ladite surface convexe est choisie dans le groupe constitué de surfaces qui sont au moins en partie coniques (51) et des parties d'une surface sphérique.

3. Inhalateur selon la revendication 1, comprenant en outre un ventilateur (60) disposé sur ledit côté en amont dudit rotor (50) pour disperser et accélérer ledit médicament vers ledit rotor.

4. Inhalateur selon la revendication 1, comprenant en outre un détecteur servant à détecter la vitesse de rotation dudit rotor (50) et une unité de commande pour commander ledit moyen d'entraînement (55) selon la vitesse de rotation dudit rotor détectée par le détecteur.

5. Inhalateur selon la revendication 3, comprenant en outre un détecteur servant à détecter la vitesse de rotation dudit rotor (50) et une unité de commande pour commander ledit moyen d'entraînement (55) selon la vitesse de rotation dudit rotor détectée par le détecteur.

6. Inhalateur selon la revendication 3, dans lequel ledit ventilateur (60) et ledit rotor (50) sont disposés coaxialement.

7. Inhalateur selon la revendication 4, dans lequel ledit ventilateur (60) et ledit rotor (50) sont disposés coaxialement.

8. Inhalateur selon la revendication 5, dans lequel ledit ventilateur (60) et ledit rotor (50) sont disposés coaxialement.

9. Inhalateur selon la revendication 3, dans lequel ledit ventilateur (60), ledit rotor (50) et ledit moyen d'entraînement (55) sont disposés coaxialement.

10. Inhalateur selon la revendication 4, dans lequel ledit ventilateur (60), ledit rotor (50) et ledit moyen d'entraînement (55) sont disposés coaxialement.

11. Inhalateur selon la revendication 5, dans lequel ledit ventilateur (60), ledit rotor (50) et ledit moyen d'entraînement (55) sont disposés coaxialement.

12. Inhalateur selon la revendication 1, comprenant en outre un couvercle (42) doté d'un évent (42a) disposé en amont dudit rotor (50) et une capsule (45) au niveau dudit évent pour contenir ledit médicament.

13. Inhalateur selon la revendication 12, comprenant en outre une aiguille partiellement ouverte (44) insérée dans ledit évent.

14. Inhalateur selon la revendication 13, dans lequel un arbre d'entraînement dudit moyen d'entraînement (55), un axe central dudit rotor (50) et ladite aiguille (44) sont disposés coaxialement.

15. Inhalateur selon la revendication 1, comprenant en outre un détecteur de vitesse d'aspiration de l'air (65) pour mesurer la vitesse d'aspiration de l'air par un patient, ledit moyen d'entraînement (55) faisant tourner le rotor (50) selon la vitesse d'aspiration de l'air mesurée par ledit détecteur de vitesse d'aspiration de l'air (65).

16. Inhalateur selon la revendication 4, comprenant en outre un détecteur de vitesse d'aspiration de l'air (65) pour mesurer la vitesse d'aspiration de l'air par un patient ; ladite unité de commande commandant ledit moyen d'entraînement (55) selon la vitesse d'aspiration de l'air mesurée par ledit détecteur de vitesse d'aspiration de l'air (65).

17. Inhalateur selon la revendication 5, comprenant en outre un détecteur de vitesse d'aspiration de l'air (65) pour mesurer la vitesse d'aspiration de l'air par un patient ; ladite unité de commande commandant ledit moyen d'entraînement (55) selon la vitesse d'aspiration de l'air mesurée par ledit détecteur de vitesse d'aspiration de l'air (65).

18. Inhalateur selon la revendication 1, dans lequel ledit rotor a une vitesse de rotation supérieure ou égale à 1000 min⁻¹.
